# EUROPEAN PATENT SPECIFICATION

(11) **EP 4 005 636 B1**
(45) Date of publication and mention of the grant of the patent: **08.01.2025**
(21) Application number: 20844574.2
(22) Date of filing: 10.07.2020
(51) Int. Cl.: A61K 33/30, A61P 19/00, A61K 47/32, A61L 27/02, A61L 27/16, A61P 19/08

(54) **BONE REGENERATIVE AGENT AND METHOD OF USING SAME**
KNOCHENREGENERATIONSMITTEL UND VERFAHREN ZU DESSEN VERWENDUNG
AGENT DE RÉGÉNÉRATION OSSEUSE ET MÉTHODE D'UTILISATION DUDIT AGENT

(30) Priority: 25.07.2019 JP 2019136934
(43) Date of publication of application: 01.06.2022
(73) Proprietor: JFE Mineral Company, Ltd., Tokyo 105-0014 (JP)
(72) Inventor: NAKATA Yoshimi, Tokyo 105-0014 (JP); UDAGAWA Etsurou, Tokyo 105-0014 (JP); YAMAMOTO Osamu, Yonezawa City, Yamagata 9928510 (JP)
(74) Representative: Grünecker Patent- und Rechtsanwälte PartG mbB
(86) International application number: PCT/JP2020/026963
(87) International publication number: WO 2021/014994

(56) References cited:
- EP-A1- 3 308 794
- WO-A1-2016/199907
- ALVES, M.M ET AL.: "In silico, in vitro and antifungal activity of the surface layers formed on zinc during this biomaterial degradation", APPL. SURF. SCI., vol. 447, 31 July 2018 (2018-07-31), pages 401 - 407, XP055786065, ISSN: 0169-4332
- ALVES, M.M ET AL.: "In vitro degradation of ZnO flowered coated Zn-Mg alloys in simulated physiological conditions", MATER. SCI. ENG. C., MATER. BIOL. APPL., vol. 70, 28 August 2016 (2016-08-28), pages 112 - 120, XP029780704, ISSN: 0928-4931, DOI: 10.1016/j.msec.2016.08.071

## Description

### TECHNICAL FIELD

The present invention relates to a bone regeneration agent as defined in the appended claims for use in a method of restoring a bone defect part.

### BACKGROUND ART

Conventionally, a bone regeneration agent containing β-tricalcium phosphate (TCP) is known (see Patent Literature 1). Alves et al., Appl. Surf. Sci., 2018, vol. 447, 401-407 disclose simonkolleite or zincite for use in bone regeneration.

### CITATION LIST

### PATENT LITERATURES

Patent Literature 1: JP 2017-61419 A

### SUMMARY OF INVENTION

### TECHNICAL PROBLEMS

The conventional bone regeneration agent (β-TCP) may sometimes remain in a bone defect part. For a living body, the remaining bone regeneration agent is a foreign substance. In bone remodeling, bone resorption (formation of resorption holes) by osteoclasts can be activated in a region around the remaining bone regeneration agent. Therefore, it is demanded that a bone regeneration agent does not remain in a bone defect part.

Accordingly, the invention has an object to provide a bone regeneration agent capable of restoring a bone defect part while minimizing residue of the agent.

### SOLUTION TO PROBLEMS

The present inventors have made an intensive study and as a result found that when the configuration described below is employed, the foregoing object is achieved. The invention has been thus completed.

Specifically, the present invention provides the following [1] to [8].
[1] A bone regeneration agent comprising a zinc carbonate hydroxide including hydrozincite for use in a method of restoring a bone defect part.
[2] The bone regeneration agent for use according to [1],
   wherein the zinc carbonate hydroxide has an amount of dissolved Zn²⁺ ions of not less than 0.1 mass ppm after a dissolution test and pH of not lower than 7.2 and lower than 8.3 after a dissolution test.
[3] The bone regeneration agent for use according to [1] or
   , wherein part of carbonate ions of the hydrozincite is substituted with sulfate ions.
[4] The bone regeneration agent for use according to [1] or
   , wherein part of carbonate ions of the hydrozincite is substituted with chloride ions.
[5] The bone regeneration agent for use according to any one
   of [1] to [4], wherein the bone regeneration agent is applied to a bone defect part.
[6] The bone regeneration agent for use according to any one of [1] to [5], further comprising a biocompatible polymer,
   wherein the bone regeneration agent is in a paste form.
[7] The bone regeneration agent for use according to [6],
   wherein the biocompatible polymer is polymethyl methacrylate.
[8] The bone regeneration agent for use according to any one of [1] to [7], comprising implanting the bone regeneration agent in a bone defect part, and covering the bone defect part with remaining periosteum.

### ADVANTAGEOUS EFFECTS OF INVENTION

According to the invention, a bone defect part can be restored while minimizing residue of the agent.

### BRIEF DESCRIPTION OF DRAWINGS

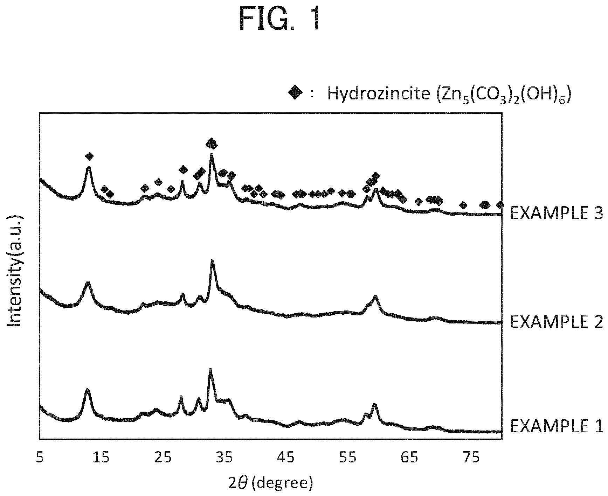

[FIG. 1] FIG. 1 is a graph showing XRD patterns of zinc carbonate hydroxides including hydrozincite of Examples 1 to 3.

### DESCRIPTION OF EMBODIMENTS

### [Bone Regeneration Agent]

The bone regeneration agent of the invention is a bone regeneration agent containing a zinc carbonate hydroxide including hydrozincite (hereinafter, also simply referred to as "zinc carbonate hydroxide").

It is conceivable that in the bone regeneration agent of the invention, zinc ions (Zn²⁺ ions) that are generated when a zinc carbonate hydroxide dissolves promote differentiation of hepatocytes in bone marrow into osteoblasts, whereby calcification of osteoblasts is induced.

### <Zinc Carbonate Hydroxide Including Hydrozincite>

Hydrozincite is expressed by, for instance, Zn₅(CO₃)₂(OH)₆ or Zn₅(CO₃)₂(OH)₆•nH₂O (where n is 0 to 6, and is preferably 2).

### <<First Embodiment>>

The zinc carbonate hydroxide including hydrozincite as described above (first embodiment) is preferably a zinc carbonate hydroxide expressed by, for example, the following Formula (1). Here, a molar ratio of Zn to CO₃, i.e., Zn/CO₃ is preferably 2.5 to 3.3.

Zn₄₋₆(CO₃)₁₋₃(OH)₅₋₆•nH₂O (1)

In Formula (1), n is 0 to 6.

The hydrozincite content in the zinc carbonate hydroxide is preferably not less than 60 mass%, more preferably not less than 80 mass%, and further preferably not less than 95 mass%.

### <<Second Embodiment>>

In the zinc carbonate hydroxide including hydrozincite (second embodiment), part of carbonate ions (CO₃²⁻) of hydrozincite may be substituted with sulfate ions (SO₄²⁻), for example. As long as the sulfur (S) content is not more than a predetermined value, its mineral phase belongs to hydrozincite.

The zinc carbonate hydroxide of the second embodiment is preferably a zinc carbonate hydroxide containing sulfur (S) in an amount of not less than 0.1 mass% and less than 1.5 mass%, and is more preferably a zinc carbonate hydroxide expressed by the following Formula (2). Here, a molar ratio of Zn to ((1-x)CO₃ + x(SO₄)), i.e., Zn/((1-x)CO₃ + x(SO₄) is preferably 2.5 to 3.3.

Zn₄₋₆((1-x)CO₃ + x(SO₄))₁₋₃(OH)₅₋₆ • nH₂O (2)

In Formula (2), n is 0 to 6, and x is 0.005 to 0.1.

### <<Third Embodiment>>

In the zinc carbonate hydroxide including hydrozincite (third embodiment), part of carbonate ions (CO₃²⁻) of hydrozincite may be substituted with chlorine ions (Cl-), for example. As long as the chloride (Cl) content is not more than a predetermined value, its mineral phase belongs to hydrozincite.

The zinc carbonate hydroxide of the third embodiment is preferably a zinc carbonate hydroxide containing chlorine (Cl) in an amount of not less than 0.05 mass% and less than 1 mass%, and is more preferably a zinc carbonate hydroxide expressed by the following Formula (3). Here, a molar ratio of Zn to ((1-x)CO₃ + xCl), i.e., Zn/((1-x)CO₃ + xCl) is preferably 2.5 to 3.3.

Zn₄₋₆((1-x)CO₃ + xCl)₁₋₃(OH)₅₋₆ • nH₂O (3)

In Formula (3), n is 0 to 6, and x is 0.005 to 0.1.

In other words, the "zinc carbonate hydroxide including hydrozincite" according to the invention (first, embodiment, second embodiment and third embodiment) can mean "zinc carbonate hydroxide including a hydrozincite-compound" as defined in the appended claims, which is collectively called "zinc carbonate hydroxide including hydrozincite" (or, simply "zinc carbonate hydroxide").

Because the residue can be further minimized, the first embodiment or the second embodiment is preferred.

Because an amount of dissolved Zn²⁺ ions after the dissolution test to be described later increases, the second embodiment or the third embodiment is preferred, and the second embodiment is more preferred.

### <<Characteristics After Dissolution Test>>

The zinc carbonate hydroxide including hydrozincite has an amount of dissolved Zn²⁺ ions of preferably not less than 0.1 mass ppm after the dissolution test and pH of preferably not lower than 7.2 and lower than 8.3 after the dissolution test.

The amount of dissolved Zn²⁺ ions after the dissolution test is more preferably not less than 0.5 mass ppm and further preferably not less than 1.0 mass ppm. Because the bone regeneration area increases, the amount of dissolved Zn²⁺ ions after the dissolution test is more preferably not less than 5.0 mass ppm, further preferably not less than 10.0 mass ppm, particularly preferably not less than 15.0 mass ppm, and most preferably not less than 20.0 mass ppm.

Meanwhile, in view of suppression of cytotoxicity, the amount of dissolved Zn²⁺ ions after the dissolution test is preferably not more than 80 mass ppm, more preferably not more than 50 mass ppm, and further preferably not more than 30 mass ppm.

The pH after the dissolution test is more preferably not higher than 8.2, further preferably not higher than 8.0, and particularly preferably not higher than 7.8.

The characteristics after the dissolution test are determined as described below.

First, a zinc carbonate hydroxide including hydrozincite is stirred in saline having temperature of 37°C at 500 rpm for 3 hours with a rotor. The concentration of the zinc carbonate hydroxide including hydrozincite in saline is 20 g/L. The dissolution test is performed as describe above.

After the dissolution test (i.e., after three hours of stirring), an amount of Zn²⁺ ions (unit: mass ppm) having been dissolved in saline is measured using an ICP emission spectrometer (ICPE-9000, manufactured by Shimadzu Corporation). The value obtained from the measurement is an amount of dissolved Zn²⁺ ions of the zinc carbonate hydroxide including hydrozincite after the dissolution test.

The saline after the dissolution test is measured for its pH, and the obtained value is the pH of the zinc carbonate hydroxide including hydrozincite after the dissolution test.

### <<Average Particle Diameter>>

The secondary particles of the zinc carbonate hydroxide contained in the bone regeneration agent of the invention have an average particle diameter of preferably 5 to 30 um, and more preferably 10 to 20 um.

The average particle diameter of the secondary particles is a particle diameter (D50) with which a cumulative frequency in a particle size distribution obtained by a laser diffraction-scattering type particle size distribution analyzer (CILAS 1064L, manufactured by CILAS) becomes 50% by volume.

### <Other Components>

The bone regeneration agent of the invention can further contain, when necessary, a pharmaceutically acceptable carrier (hereinafter, simply referred to as "carrier").

Exemplary carriers include solvents such as an organic solvent and an inorganic solvent, and specific examples thereof include water, saline, alcohols, polyhydric alcohols, and mixtures thereof.

For instance, in a case where the bone regeneration agent of the invention contains a solvent as a carrier, a thickener or the like may be further added to the bone regeneration agent of the invention, whereby the bone regeneration agent of the invention may be processed into a gel form or a paste form to improve its handleability.

An exemplary thickener is a biocompatible polymer, and specific examples thereof include polymethyl methacrylate (PMMA).

In addition to the foregoing, for example, carriers described in paragraphs [0027] to [0030] of WO2016/199907 may be used as the carrier.

Depending on an actual application, other additives may be contained in the bone regeneration agent of the invention.

Other additives are not particularly limited, and examples thereof include humectants, antioxidants, preservatives, antiphlogistic agents, whitening agents, blood circulation promoting agents, antiseborrheic agents, thickeners, and pH adjusters. Specific examples thereof include components described in paragraphs [0031] to [0034] of WO2016/199907.

The bone regeneration agent of the invention may take a form of liquid containing a carrier such as saline as a solvent, or a form of powder free from a solvent (carrier). Moreover, the bone regeneration agent may take a form between the foregoing forms (e.g., paste form) or a gelated form.

The references to the methods of treatment by therapy or surgery in this paragraph are to be interpreted as references to compounds, pharmaceutical compositions and medicaments of the present invention for use in those methods.

### [Method of Using Bone Regeneration Agent]

An exemplary method of using the bone regeneration agent of the invention is a method in which the bone regeneration agent of the invention is implanted (filled) in a bone defect part. At this time, the site where the bone regeneration agent of the invention is implanted (implanted site) may be covered with remaining periosteum or the like.

After the bone regeneration agent of the invention is implanted in a bone defect part, a predetermined period of time is allowed to elapse.

Accordingly, tissues at the bone defect part regenerate, thereby turning to a new bone part. In other words, the bone defect part is restored. In this process, the bone regeneration agent of the invention hardly remains at the bone defect part.

### [Method of Producing Bone Regeneration Agent]

An exemplary method of producing the bone regeneration agent of the invention is a method in which a zinc carbonate hydroxide including hydrozincite is obtained, and as necessary, a carrier or the like is combined therewith.

The zinc carbonate hydroxide including hydrozincite is preferably obtained through a precipitate forming reaction as described below.

### <Precipitate Forming Reaction>

The zinc carbonate hydroxide including hydrozincite is preferably obtained through a precipitate forming reaction (alkaline precipitation process) using a zinc source, a carbonic acid source and alkali.

Examples of zinc sources include zinc sulfate (ZnSO₄), zinc chloride (ZnCl₂), zinc acetate (Zn(CH₃COO)₂), and zinc nitrate (Zn(NO₃)₂).

For obtaining the zinc carbonate hydroxide of the first embodiment, zinc nitrate is preferably used.

For obtaining the zinc carbonate hydroxide of the second embodiment, zinc sulfate is preferably used.

For obtaining the zinc carbonate hydroxide of the third embodiment, zinc chloride is preferably used.

Examples of carbonic acid sources include ammonium carbonate ((NH₄)₂CO₃), sodium carbonate (Na₂CO₃), and sodium hydrogen carbonate (NaH(CO₃)), and sodium hydrogen carbonate is preferred.

For obtaining the zinc carbonate hydroxide of the second embodiment, ammonium sulfate can be used in combination with a carbonic acid source.

For obtaining the zinc carbonate hydroxide of the third embodiment, ammonium chloride can be used in combination with a carbonic acid source.

Examples of alkali include ammonia (NH₃), and sodium hydroxide (NaOH), and sodium hydroxide is preferred.

It is preferable that a zinc source, a carbonic acid source and alkali are used in the form of aqueous solution.

In the precipitate forming reaction, as a specific example, it is preferable that an aqueous zinc source solution (aqueous acid solution) is added dropwise to an aqueous carbonic acid source solution, while an aqueous solution of alkali is supplied to the aqueous carbonic acid source solution to keep the pH of the aqueous carbonic acid source solution within a certain range, and after dropwise addition of the aqueous zinc source solution is terminated, the resulting solution mixture is stirred (stirred and cured) for 10 to 30 hours, whereby a reaction solution containing a precipitate is obtained.

The pH can vary depending on a type, a concentration and the like of the carbonic acid source and the zinc source used and is preferably not lower than 6.5 and lower than 9.5, and more preferably not lower than 7.0 and lower than 9.5. In other words, while a precipitate is obtained through a reaction of Zn²⁺ ions, CO₃²⁻ ions and OH- ions (and SO₄²⁻ ions, Cl- ions or the like are further contained in some cases), it is preferable to use a precipitate obtained in a reaction field where the pH is controlled to be within the foregoing range.

A molar ratio of zinc to carbonic acid (zinc:carbonic acid) of the zinc source and the carbonic acid source is preferably 5:2.

In the second embodiment and the third embodiment, the molar ratio of zinc to anions (zinc:anions) is preferably 5:2. The molar ratio in the anions (i.e., CO₃²⁻ :SO₄²⁻ or CO₃²⁻:Cl⁻) is appropriately set in view of the S content or the Cl content in the obtained zinc carbonate hydroxide.

The concentration of the aqueous zinc source solution is preferably not less than 0.01 mol/L and more preferably not less than 0.03 mol/L. Meanwhile, the concentration is preferably not more than 3 mol/L and more preferably not more than 1 mol/L.

The concentration of the aqueous carbonic acid source solution is preferably not less than 0.004 mol/L and more preferably not less than 0.012 mol/L. Meanwhile, the concentration is preferably not more than 1.2 mol/L and more preferably not more than 0.4 mol/L.

The reaction temperature is preferably not lower than 15°C. Meanwhile, the temperature is preferably not higher than 60°C and more preferably not higher than 40°C.

Following the precipitation forming reaction, for example, the reaction solution containing a precipitate is subjected to suction filtration or centrifugation to be separated into solid and liquid, and the obtained precipitate is washed with pure water or distilled water and subsequently dried in vacuum. Zinc carbonate hydroxide powder is thus obtained. Thereafter, an appropriate particle diameter of the powder may be attained by a known method.

The obtained zinc carbonate hydroxide powder can contain an unreacted material (raw material), a reaction byproduct, an impurity from the raw materials, and other substances.

### EXAMPLES

Hereinafter, the invention is specifically described by way of examples. However, the present invention is not limited thereto.

### <Example 1>

In a reaction vessel, 0.08 mol/L aqueous sodium hydrogen carbonate solution (500 mL) was prepared. Separately, 0.1 mol/L aqueous zinc nitrate solution (1,000 mL) was prepared. Further, as a pH adjusting liquid, 30 mass% aqueous sodium hydroxide solution was prepared.

A pH electrode connected to a pH controller was provided in the aqueous sodium hydrogen carbonate solution in the reaction vessel. Using a pump that is turned on and off with the pH controller, the aqueous zinc nitrate solution and the aqueous sodium hydroxide solution were added dropwise to the aqueous sodium hydrogen carbonate solution. During the dropwise addition, the pH of the aqueous sodium hydrogen carbonate solution was kept at 7.0. During the dropwise addition, the aqueous sodium hydrogen carbonate solution was stirred with a rotor. After all of the aqueous zinc nitrate solution was added dropwise, the solution mixture was further stirred for 16 hours and cured. The reaction temperature (environmental temperature during the dropwise addition and the curing) was 25°C. A reaction solution containing a precipitate was obtained in this manner.

The obtained reaction solution was separated into solid and liquid by centrifugation. The obtained solid (precipitate) was repeatedly subjected to water washing and centrifugation three times so as to be washed. The precipitate thus washed was dried in vacuum, whereby the zinc carbonate hydroxide powder of the first embodiment as described above was obtained. The obtained powder was treated as the bone regeneration agent of Example 1.

The zinc carbonate hydroxide powder obtained in Example 1 was subjected to XRD measurement using an XRD device (D8 ADVANCE manufactured by Bruker Corporation) (the same measurement was performed also in Example 2 and Example 3 described later). The result of the XRD measurement is shown in FIG. 1. In the XRD pattern shown in FIG. 1, peaks representing hydrozincite are recognized.

### <Example 2>

The zinc carbonate hydroxide powder of the second embodiment as described above was obtained in the same manner as in Example 1 except that 0.1 mol/L aqueous zinc sulfate solution (1,000 mL) was used in place of 0.1 mol/L aqueous zinc nitrate solution (1,000 mL). The obtained powder was treated as the bone regeneration agent of Example 2. In the XRD pattern shown in FIG. 1, peaks representing hydrozincite are recognized.

### <Example 3>

The zinc carbonate hydroxide powder of the third embodiment as described above was obtained in the same manner as in Example 1 except that 0.1 mol/L aqueous zinc chloride solution (1,000 mL) was used in place of 0.1 mol/L aqueous zinc nitrate solution (1,000 mL). The obtained powder was treated as the bone regeneration agent of Example 3. In the XRD pattern shown in FIG. 1, peaks representing hydrozincite are recognized.

### <Characteristics After Dissolution Test>

Of the zinc carbonate hydroxide powders (bone regeneration agents) of Example 1 to Example 3, amounts of dissolved Zn²⁺ ions and the pH after the dissolution test were determined according to the foregoing methods. The results are shown in Table 1 below.

### [Table 1]

**Table 1**

| | Example 1 | Example 2 | Example 3 |
|---|---|---|---|
| Amount of dissolved Zn²⁺ ions after dissolution test [mass ppm] | 1.3 | 25.7 | 6.1 |
| pH after dissolution test | 7.8 | 7.2 | 7.5 |

### <EVALUATION>

The zinc carbonate hydroxide powder (bone regeneration agent) of Example 2 was used in an animal testing.

In addition, as a bone regeneration agent of Comparative Example 1, a commercially available bone filling material, β-TCP (OSferion (registered trademark), manufactured by Olympus Terumo Biomaterials Corporation) was used.

In the animal testing, the bone regeneration agent was implanted in a defect part of a rib in a Zen-noh Premium pig. The Zen-noh Premium pig was under full anesthesia, and local anesthesia was given at the implanted site using a dental anestheric, Xylocaine.

More specifically, of the Zen-noh Premium pig, hairs in left and right sides of the ventral part were shaved, a rib was exposed, and a bone defect part of about 2 mm x 14 mm was made in the exposed rib using Volvere Vmax. The bone regeneration agent in an amount of 0.005 g was implanted in the bone defect part, the defect part was covered with remaining periosteum, and thereafter the wound was closed. The implantation period was four weeks.

After four weeks have elapsed, the Zen-noh Premium pig was anesthetized with isoflurane and was bled, and subsequently part of the rib (the implanted site and its peripheral part) was extracted. Soft tissues attached to the extracted rib were trimmed, and bone regeneration was then evaluated.

### <<Macroscopic Evaluation>>

The implanted site (bone defect part) of the rib thus extracted was observed using a system microscope (BX53, manufactured by Olympus Corporation).

As a result, in Comparative Example 1, while bone regeneration in the bone defect part was confirmed, a large amount of the bone regeneration agent (β-TCP) was remaining when the bone defect part was viewed in cross section.

In Example 2, on the other hand, bone regeneration in the bone defect part was confirmed, and no residue of the bone regeneration agent (zinc carbonate hydroxide) was recognized.

### <<Histological Evaluation>>

The implanted site (bone defect part) of the rib thus extracted was hematoxylin and eosin (HE) stained and Masson's trichrome (MT) stained.

As a result, in Comparative Example 1, while the bone defect part implanted with the bone regeneration agent (β-TCP) was calcified, a number of resorption holes were present, and inflammatory cells were recognized.

In Example 2, on the other hand, no residue of the bone regeneration agent (zinc carbonate hydroxide) in the bone defect part was recognized, and a thick calcified bone was formed.

Also in the cases where the bone regeneration agents of Example 1 and Example 3 were used, the similar results as those of the case where the bone regeneration agent of Example 2 was used were obtained. In the meantime, while a very small amount of the bone regeneration agent of Example 3 was remained, no residue of the bone regeneration agent of Example 1 or Example 2 was observed.

There was also a difference in the bone regeneration area. In particular, the area of tissue regeneration in the bone defect part was larger in Example 2, Example 3, Example 1, and Comparative Example 1 in this order (Example 2 yielded the largest bone regeneration area).

### <Summary of Evaluation Results>

The foregoing results revealed that while the bone regeneration agent (β-TCP) of Comparative Example 1 remained in the defect part of the rib, the bone regeneration agents of Examples 1 to 3 could restore the defect part of the rib with their residues being minimized.

Since a zinc carbonate hydroxide including hydrozincite dissolves when contacting a body fluid, all of the zinc carbonate hydroxide including hydrozincite have probably dissolved in a rib which was abundant with bone marrow. Therefore, zinc carbonate hydroxide including hydrozincite is suitable for a bone regeneration agent.

## Claims

1. A bone regeneration agent comprising a zinc carbonate hydroxide including hydrozincite for use in a method of restoring a bone defect part.

2. The bone regeneration agent for use according to claim 1, wherein the zinc carbonate hydroxide has an amount of dissolved Zn²⁺ ions of not less than 0.1 mass ppm after a dissolution test and pH of not lower than 7.2 and lower than 8.3 after a dissolution test.

3. The bone regeneration agent for use according to claim 1 or 2, wherein part of carbonate ions of the hydrozincite is substituted with sulfate ions.

4. The bone regeneration agent for use according to claim 1 or 2, wherein part of carbonate ions of the hydrozincite is substituted with chloride ions.

5. The bone regeneration agent for use according to any one of claims 1 to 4, further comprising a biocompatible polymer,
wherein the bone regeneration agent is in a paste form.

6. The bone regeneration agent for use according to claim 5, wherein the biocompatible polymer is polymethyl methacrylate.

7. The bone regeneration agent for use according to any one of claims 1 to 6, comprising implanting the bone regeneration agent in a bone defect part, and covering the bone defect part with remaining periosteum.

## Patentansprüche

1. Knochenregenerationsmittel, umfassend ein Zinkcarbonathydroxid einschließlich Hydrozinkit zur Verwendung in einem Verfahren zur Wiederherstellung eines Knochendefektteils.

2. Knochenregenerationsmittel zur Verwendung gemäß Anspruch 1, wobei das Zinkcarbonathydroxid eine Menge an gelösten Zn²⁺-Ionen von nicht weniger als 0,1 Massen-ppm nach einem Auflösungstest und einen pH-Wert von nicht weniger als 7,2 und weniger als 8,3 nach einem Auflösungstest aufweist.

3. Knochenregenerationsmittel zur Verwendung nach Anspruch 1 oder 2, wobei ein Teil der Carbonationen des Hydrozinkits durch Sulfationen ersetzt ist.

4. Knochenregenerationsmittel zur Verwendung nach Anspruch 1 oder 2, wobei ein Teil der Carbonationen des Hydrozinkits durch Chloridionen ersetzt ist.

5. Knochenregenerationsmittel zur Verwendung nach einem der Ansprüche 1 bis 4, ferner umfassend ein biokompatibles Polymer,
wobei das Knochenregenerationsmittel in einer Pastenform vorliegt.

6. Knochenregenerationsmittel zur Verwendung nach Anspruch 5, wobei das biokompatible Polymer Polymethylmethacrylat ist.

7. Knochenregenerationsmittels zur Verwendung nach einem der Ansprüche 1 bis 6, umfassend das Implantieren des Knochenregenerationsmittels in einen Knochendefektteil und das Abdecken des Knochendefektteils mit verbleibender Knochenhaut.

## Revendications

1. Agent de régénération osseuse comprenant un hydroxyde de carbonate de zinc incluant de l'hydrozincite pour l'utilisation dans un procédé de restauration d'une partie défectueuse d'un os.

2. Agent de régénération osseuse pour l'utilisation selon la revendication 1, dans lequel l'hydroxyde de carbonate de zinc présente une quantité d'ions Zn²⁺ dissous non inférieure à 0,1 ppm en masse après un test de dissolution et un pH non inférieur à 7,2 et inférieur à 8,3 après un test de dissolution.

3. Agent de régénération osseuse pour l'utilisation selon la revendication 1 ou 2, dans lequel une partie des ions carbonates de l'hydrozincite est substituée par des ions sulfates.

4. Agent de régénération osseuse pour l'utilisation selon la revendication 1 ou 2, dans lequel une partie des ions carbonates de l'hydrozincite est substituée par des ions chlorures.

5. Agent de régénération osseuse pour l'utilisation selon l'une quelconque des revendications 1 à 4, comprenant en outre un polymère biocompatible,
dans lequel l'agent de régénération osseuse se présente sous la forme d'une pâte.

6. Agent de régénération osseuse pour l'utilisation selon la revendication 5, dans lequel le polymère biocompatible est le poly(méthacrylate de méthyle).

7. Agent de régénération osseuse pour l'utilisation selon l'une quelconque des revendications 1 à 6, comprenant l'implantation de l'agent de régénération osseuse dans une partie défectueuse d'un os, et le recouvrement de la partie défectueuse d'un os avec le périoste restant.
